# DEMANDE DE BREVET EUROPEEN

(11) **EP 1 323 756 A1**
(43) Date de publication de la demande: **02.07.2003**
(21) Numéro de dépôt: 02293168.7
(22) Date de dépôt: 19.12.2002
(51) Int. Cl.: C08G 18/08, C08G 18/48, C08G 18/66, C08G 18/76, A61K 7/06

(54) **Polyuréthannes cationiques ou amphotères auto-adhésifs**

(30) Priorité: 20.12.2001 FR 0116598
(71) Demandeur: L'OREAL, 75008 Paris (FR)
(72) Inventeur: Perron, Béatrice, 78350 Jouy en Josas (FR); Resile, Serge, 95390 Saint Prix (FR); Mougin, Nathalie, 75011 Paris (FR)
(74) Mandataire: Casalonga, Axel

(57) **Abrégé**

L'invention concerne des polyuréthannes cationiques ou amphotères portant au moins une fonction amine tertiaire ou quaternaire et présentant une auto-adhésivité - exprimée par la force de traction maximale (Fₘₐₓ (en N)) enregistrée lors du décollement par traction de deux surfaces circulaires de 0,95 cm², enduites desdits polyuréthannes - supérieure ou égale à 11 N. Elle concerne également l'utilisation de ces polyuréthannes cationiques auto-adhésifs en cosmétique et en particulier dans des compositions de coiffage.

## Description

La présente invention concerne de nouveaux polyuréthannes cationiques ou amphotères auto-adhésifs, l'utilisation de ces polyuréthannes auto-adhésifs en cosmétique, ainsi que des compositions cosmétiques, et en particulier des compositions de coiffage, les contenant.

Les polymères cationiques hydrosolubles, tels que les polymères à base de chlorure de diméthyldiallylammonium, sont utilisés depuis longtemps dans le domaine cosmétique, et en particulier dans le domaine des soins capillaires. En effet, leur bonne affinité (substantivité) pour les substrats kératiniques et en particulier leur capacité à former des films continus autour des cheveux en font d'excellents candidats pour protéger, embellir et renforcer les cheveux.

Cependant, en raison de leur viscosité élevée et de leur incompatibilité avec la plupart des agents propulseurs, les polymères de cette famille sont difficiles à utiliser dans des produits aérosol tels que des laques.

Les polymères cationiques couramment utilisés dans le domaine des soins capillaires présentent par ailleurs une faible auto-adhésivité, c'est-à-dire les fibres capillaires entourées d'une gaine de ces polymères cationiques n'adhèrent que peu ou pas du tout les unes aux autres.

La demanderesse a découvert une nouvelle famille de polyuréthannes cationiques ou amphotères particuliers, présentant une auto-adhésivité élevée et qui possèdent une substantivité suffisante et un très bon pouvoir coiffant. Cette association de propriétés les rend particulièrement appropriés pour une utilisation dans des compositions de coiffage à rincer telles que des shampooings coiffants.

Bien entendu, leur utilisation dans des produits coiffants non-rincés est également avantageuse car ces polyuréthannes cationiques ou amphotères auto-adhésifs peuvent alors être utilisés en des quantités nettement plus faibles que les polymères cationiques ou amphotères connus ou les polymères auto-adhésifs anioniques ou neutres. La possibilité d'utiliser les polymères de la présente invention en de faibles quantités facilite leur formulation et réduit la viscosité des compositions obtenues.

Les polyuréthannes cationiques ou amphotères auto-adhésifs de la présente invention peuvent également être utilisés dans des domaines cosmétiques autres que celui du coiffage. Ainsi, l'introduction de faibles quantités de ces polyuréthannes dans la plupart des produits de maquillage assure une bonne adhérence des dépôts cosmétiques sur la peau et leur confère une bonne cohésivité et souplesse. Le maquillage ne craquèle pas et ne tire pas la peau des utilisateurs.

L'invention a par conséquent pour objet des polyuréthannes cationiques ou amphotères portant au moins une fonction amine tertiaire ou quaternaire et présentant une auto-adhésivité - exprimée par la force de traction maximale (Fₘₐₓ (en N)) enregistrée lors du décollement par traction de deux surfaces circulaires de 0,95 cm², enduites desdits polyuréthannes - supérieure ou égale à 11 N.

L'invention a également pour objet des compositions cosmétiques, et en particulier des compositions de coiffage, contenant, dans un milieu cosmétiquement acceptable, au moins un tel polyuréthanne cationique ou amphotère auto-adhésif.

L'invention a également pour objet l'utilisation en cosmétique des nouveaux polyuréthannes cationiques ou amphotères auto-adhésifs décrits ci-dessus.
L'invention a enfin pour objet un procédé de traitement des matières kératiniques comprenant l'application sur les matières kératiniques d'une composition cosmétique telle que décrite ci-dessus, ainsi qu'un procédé de coiffage comprenant l'application d'une telle composition cosmétique sur les cheveux, le rinçage des cheveux, puis la mise en forme et le séchage des cheveux rincés.

Le caractère auto-adhésif des polyuréthannes cationiques ou amphotères de la présente invention est évalué selon le protocole suivant :
On dépose sur la surface de deux plaquettes circulaires en verre dépoli, ayant chacune une surface de 0,95 cm² (diamètre 11 mm), 40 µl d'une solution ou dispersion aqueuse contenant 10 % en poids de polymère à tester. On laisse sécher pendant 48 heures à pression ambiante, à un taux d'humidité relative de 55 % et à une température de 22 °C.
Les deux plaquettes sont fixées dans un appareil de mesure de la résistance en traction (Lloyd LR5K) et sont pressées l'une contre l'autre pendant 20 secondes avec une force de 3 N. On sépare ensuite, dans les mêmes conditions de température et d'humidité relative, les deux plaquettes pendant 30 secondes en imposant une vitesse de traction de 20 mm/minute, et l'on enregistre la force nécessaire à ce déplacement, et plus particulièrement la force maximale (Fₘₐₓ) en Newton (N) mesurée au moment de la séparation brusque des deux surfaces revêtues de polymère. Bien entendu, l'auto-adhésivité des polymères de la présente invention est d'autant plus forte que la force maximale enregistrée est élevée.

Dans un mode de réalisation préféré de la présente invention, les polyuréthannes cationiques de la présente invention comprennent
(a) des motifs dérivés d'une ou de plusieurs amines tertiaires ou quaternaires comportant deux fonctions réactives à hydrogène labile,
(b) des motifs dérivés d'un ou de plusieurs polymères non ioniques comportant deux fonctions réactives à hydrogène labile, et
(c) des motifs dérivés d'un ou de plusieurs diisocyanates.

Les amines tertiaires ou quaternaires formant les motifs cationiques (a) sont de préférence choisies parmi les composés correspondant à l'une des formules suivantes : 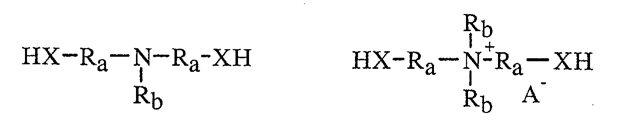 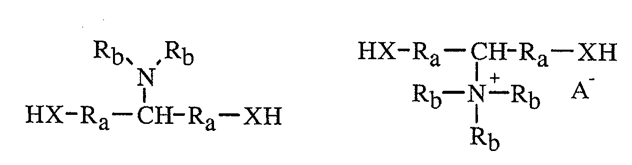 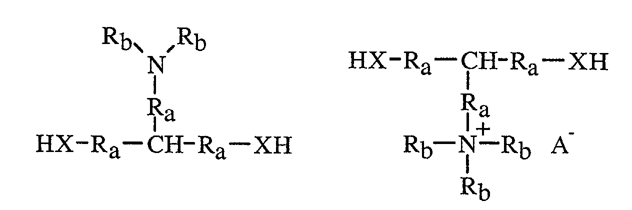 dans lesquelles
chaque Rₐ représente indépendamment un groupe alkylène en C₁₋₆, linéaire ou ramifié, cycloalkylène en C₃₋₆ ou arylène, tous pouvant être substitués par un ou plusieurs atomes d'halogène et comporter un ou plusieurs hétéroatomes choisis parmi O, N, P et S,
chaque R_{b} représente indépendamment un groupe alkyle en C₁₋₆, cycloalkyle en C₃₋₆ ou aryle, tous pouvant être substitués par un ou plusieurs atomes d'halogène et comporter un ou plusieurs hétéroatomes choisis parmi O, N, P et S,
chaque X représente indépendamment un atome d'oxygène ou de soufre ou un groupe NH ou NR_{c}, où R_{c} représente un groupe alkyle en C₁₋₆, et
A⁻ représente un contre-ion physiologiquement acceptable.

On peut citer à titre d'exemples d'amines tertiaires particulièrement préférées pour l'obtention des polyuréthannes cationiques ou amphotères auto-adhésifs de la présente invention la N-méthyldiéthanolamine et la N-tert-butyldiéthanolamine. Ces amines sont de préférence partiellement ou totalement neutralisées par des acides minéraux ou organiques tels que l'acide chlorhydrique ou l'acide citrique.

Les polyuréthannes auto-adhésifs de la présente invention peuvent également comporter des motifs anioniques (d) dérivés par exemple d'acides carboxyliques ou sulfoniques comportant deux fonctions à hydrogène labile, tels que l'acide diméthylolpropionique.

Les polyuréthannes auto-adhésifs de la présente invention peuvent également comporter des motifs monomères non ioniques (e) dérivés de composés monomères non ioniques comportant deux fonctions à hydrogène labile, tels que le butanediol ou le néopentylglycol.

Dans un mode de réalisation de la présente invention, les polyuréthannes autoadhésifs de la présente invention sont des polyuréthannes auto-adhésifs cationiques ne contenant pas de motifs (d) et (e) et qui sont constitués essentiellement
(a) de motifs dérivés d'une ou de plusieurs amines tertiaires ou quaternaires comportant deux fonctions réactives à hydrogène labile,
(b) de motifs dérivés d'un ou de plusieurs polymères non ioniques comportant deux fonctions réactives à hydrogène labile, et
(c) de motifs dérivés d'un ou de plusieurs diisocyanates.

La demanderesse a constaté que les polyuréthannes cationiques avaient des propriétés d'auto-adhésivité particulièrement intéressantes lorsque le ou les polymères formant les motifs (b) des polyuréthannes auto-adhésifs de la présente invention avaient une température de transition vitreuse (Tg), déterminée par analyse calorimétrique différentielle, inférieure à 0 °C, de préférence inférieure à -5 °C et mieux encore inférieure à -10 °C.

On peut indiquer à titre d'exemples de polymères non ioniques susceptibles de former les motifs (b) les polyéthers, les polyesters, les polysiloxanes, les copolymères d'éthylène et de butylène, les polycarbonates et les polymères fluorés ayant une température de transition vitreuse inférieure à 0°C.

On préfère tout particulièrement les polyéthers et parmi ceux-ci le poly(tétraméthylène oxyde).
Ces polymères ont de préférence une masse molaire moyenne en poids comprise entre 400 et 10 000 et plus particulièrement entre 500 et 5000.

Le nombre de charges cationiques portées par les polyuréthannes autoadhésifs de la présente invention dépend directement du rapport molaire ou pondéral des motifs (a) aux motifs (b). Bien entendu, les motifs (c) sont utilisés en une quantité pratiquement équimolaire par rapport à la somme des motifs (a) et (b).
Le rapport molaire des motifs (a) aux motifs (b) des polyuréthannes de la présente invention est de préférence compris entre 0,01 et 50, plus particulièrement entre 0,1 et 6, mieux encore entre 0,2 et 5 et idéalement entre 0,3 et 5.

Les diisocyanates formant les motifs (c) englobent les diisocyanates aliphatiques, alicycliques ou aromatiques.
Des diisocyanates préférés sont choisis parmi le tétraméthylxylylènediisocyanate, le méthylènediphényldiisocyanate, le méthylènecyclohexanediisocyanate, l'isophoronediisocyanate, le toluènediisocyanate, le naphtalène-diisocyanate, le butanediisocyanate et l'hexyldiisocyanate. Ces diisocyanates peuvent bien entendu être utilisés seuls ou sous forme de mélange de deux ou plusieurs diisocyanates.

Un paramètre important permettant de sélectionner, parmi les polyuréthannes cationiques ou amphotères, ceux ayant des propriétés d'auto-adhésivité avantageuses, est la température de transition vitreuse (Tg) du polyuréthanne cationique final.
En effet, les polyuréthannes cationiques ou amphotères auto-adhésifs présentent de préférence au moins une température de transition vitreuse inférieure à la température ambiante (20 °C), c'est-à-dire à température ambiante, une partie du polymère est à l'état caoutchoutique et non à l'état vitreux. Les caractéristiques d'auto-adhésivité des polymères de la présente invention sont particulièrement intéressantes lorsque la température de transition vitreuse est inférieure à 0 °C et en particulier inférieure à -20 °C.
Les polyuréthannes cationiques ou amphotères auto-adhésifs de la présente invention peuvent également présenter plusieurs températures de transition vitreuse. Lorsque ceci est le cas, les indications ci-dessus concernant la température de transition vitreuse se rapportent à la température de transition vitreuse la plus basse du polymère.

La température de transition vitreuse des polymères de la présente invention est mesurée par analyse calorimétrique différentielle (en anglais *Differential Scanning Calorimetry*, DSC) dans les conditions suivantes :
Pour mesurer la température de transition vitreuse, on réalise un film ayant une épaisseur d'environ 150 mm du polymère à tester en déposant une solution ou une dispersion aqueuse du polymère dans une matrice circulaire en téflon de 40 mm de diamètre et en laissant sécher le dépôt. Le film est mis à sécher dans une étuve à une température d'environ 23 °C sous une humidité realtive de 45 %, jusqu'à ce que le poids ne varie plus. On prélève environ 5 à 15 mg du film, que l'on place dans un creuset qui est introduit ensuite dans l'analyseur. L'analyseur thermique est un modèle DSC-2920 de la société TA INSTRUMENTS. Les températures initiales et finales du balayage en température sont choisies de manière à encadrer la température de transition vitreuse recherchée. Le balayage en température se fait à une vitesse de 10 °C/minute.
Cette analyse est effectuée selon la norme ASTM D 3418-97 aux modifications ci-dessus près.

Les polyuréthannes auto-adhésifs de la présente invention sont préparés selon des méthodes connues de polycondensation. Ces méthodes sont notamment décrites dans les ouvrages suivants:
- *60 Years of PUR -* J.E. Kresta, E.W. Eldred Ed. Technomic Publishing, 1998,
- *Waterborne and Suivent Based Surface Coating Resins and Their Application*, Série Surface Coating Technology, Vol. 3, Polyurethanes, Paul Thomas, Wiley and Sons, 1998.

Les polyuréthannes auto-adhésifs de la présente invention peuvent se présenter sous forme de solutions ou de dispersions aqueuses ou huileuses.

Comme indiqué ci-dessus, les polyuréthannes cationiques ou amphothères autoadhésifs de la présente invention peuvent être utilisés en cosmétique sous forme de compositions de soin ou de maquillage de la peau ou des phanères, en particulier sous forme de compositions de soin, de conditionnement, de maquillage ou de fixation des matières kératiniques humaines telles que les cheveux et les cils.
De préférence, ces compositions cosmétiques contiennent, dans un milieu aqueux cosmétiquement acceptable, de 0,01 % à 40 %, en particulier de 0,05 à 20 % et idéalement de 0,1 à 10 % en poids d'au moins un polyuréthanne cationique auto-adhésif de la présente invention.
Dans un mode de réalisation préféré de la présente invention, les compositions cosmétiques sont des compositions de coiffage, et en particulier des compositions de coiffage à rincer, notamment des shampooings coiffants.
Le milieu aqueux cosmétiquement acceptable peut contenir différents adjuvants et solvants couramment utilisés dans le domaine cosmétique tels que des agents tensioactifs, des polymères anioniques, amphotères, zwitterioniques ou non ioniques, des polymères cationiques différents des polyuréthannes cationiques de la présente invention, des agents nacrants et/ou opacifiants, des solvants organiques, des parfums, des huiles minérales, végétales et/ou synthétiques, des esters d'acides gras, des pigments et colorants, des silicones, des particules minérales ou organiques, des agents de stabilisation du pH, des agents conservateurs et des agents absorbant les UV.

Les tensioactifs utilisables dans la composition selon la présente invention peuvent être des tensioactifs anioniques, non ioniques, amphotères ou cationiques, ou des mélanges de ceux-ci.
Parmi les tensioactifs anioniques utilisables, seuls ou en mélanges, dans le cadre de la présente invention, on peut notamment citer les sels, et en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les alkylsulfates, les alkyléthersulfates, les alkylamidoéthersulfates, les alkylaryl-polyéthersulfates, les monoglycéride-sulfates; les alkylsulfonates, les alkylamidesulfonates, les alkyl-arylsulfonates, les α-oléfine-sulfonates, les paraffine-sulfonates ; les alkylsulfosuccinates, les alkyléthersulfosuccinates, les alkylamide-sulfosuccinates ; les alkylsulfoacétates ; les acylsarcosinates ; et les acylglutamates, les groupes alkyle et acyle de tous ces composés comportant de 6 à 24 atomes de carbone et le groupe aryle désignant de préférence un groupe phényle ou benzyle.
On peut également utiliser dans le cadre de la présente invention les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone.
En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.
Parmi les tensioactifs anioniques cités ci-dessus, on préfère utiliser selon l'invention les alkyl(C₆-C₂₄)sulfates, les alkyl(C₆-C₂₄)éthersulfates, les alkyl(C₆-C₂₄)éthercarboxylates et leurs mélanges, par exemple le laurylsulfate d'ammonium, le laurylsulfate de sodium, le laurylsulfate de magnésium, le lauryléthersulfate de sodium, le lauryléthersulfate d'ammonium et le lauryléthersulfate de magnésium.
La composition selon la présente invention peut comprendre les agents tensioactifs anioniques en une quantité comprise de préférence entre 0,5 et 60 % en poids, mieux encore entre 5 et 20 % en poids par rapport au poids total de la composition.

Les agents tensioactifs non-ioniques que l'on peut utiliser dans le cadre de la présente invention, sont eux aussi, des composés bien connus en soi (voir notamment à cet égard "Handbook of Surfactants" par M.R. PORTER, éditions Blackie & Son (Glasgow and London), 1991, pp 116-178). Ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et d'oxyde de propylène, les condensats d'oxyde d'éthylène et d'oxyde de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglucosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)aminopropylmorpholine ; et leurs mélanges.
Parmi les tensioactifs non-ioniques cités ci-dessus, on utilise de préférence les alkyl(C₆-C₂₄)polyglycosides, en particulier le décylpolyglucoside.
Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant, au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL®, tels que décrits dans les brevets US 2 528 378 et US 2 781 354 et classés dans le dictionnaire CTFA, 3ème édition, 1982, sous les dénominations Amphocarboxy-glycinate et Amphocarboxypropionate de structures respectives (1) et (2) :

R₂-CONHCH₂CH₂-N⁺(R₃)(R₄)(CH₂COO⁻) (1)

dans laquelle :
R₂ représente un groupe alkyle dérivé d'un acide R₂-COOH présent dans l'huile de coprah hydrolysée, un groupe heptyle, nonyle ou undécyle,
R₃ représente un groupe bêta-hydroxyéthyle, et
R₄ représente un groupe carboxyméthyle ; et

   R₂'-CONHCH₂CH₂-N(B)(C) (2)

   dans laquelle :
   B représente -CH₂CH₂OX',
   C représente -(CH₂)_{z}-Y', avec z = 1 ou 2,
   X' représente le groupe -CH₂CH₂-COOH ou un atome d'hydrogène,
   Y' représente -COOH ou le groupe -CH₂-CHOH-SO₃H,
   R₂' représente le groupe alkyle d'un acide R₂'-COOH présent dans l'huile de coprah ou dans l'huile de lin hydrolysée, un groupe alkyle, notamment en C₁₇ et sa forme iso, un groupe en C₁₇ insaturé.
Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.
A titre d'exemple, on peut citer le cocoamphodiacétate commercialisé sous la dénomination commerciale MIRANOL® C2M concentré par la société RHODIA.
Parmi les tensioactifs amphotères, on utilise de préférence les alkyl(C₈-C₂₀)bétaïnes telles que la cocobétaïne, les alkyl(C₈-C₂₀)amidoalkyl(C₆-C₈)bétaïnes telles que la cocamidobétaïne, les alkylamphodiacétates comme le cocoamphodiacétate disodique, et leurs mélanges.
La composition selon l'invention peut comprendre en outre un ou plusieurs tensioactifs cationiques bien connus en soi, tels que les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.
Les agents tensioactifs non ioniques, amphotères et cationiques décrits ci-dessus peuvent être utilisés seuls ou en mélanges et leur quantité est comprise entre 0,1 % et 30 % en poids, de préférence entre 0,5 % et 25 % en poids et mieux encore entre 1 % et 20 % en poids par rapport au poids total de la composition.

Les silicones utilisables en tant qu'additifs dans les compositions cosmétiques de la présente invention, sont des silicones volatiles ou non volatiles, cycliques, linéaires ou ramifiées, modifiées ou non par des groupements organiques, ayant une viscosité de 5.10⁻⁶ à 2,5 m²/s à 25°C et de préférence 1.10⁻⁵ à 1 m²/s.
Les silicones utilisables conformément à l'invention peuvent être solubles ou insolubles dans la composition et en particulier être des polyorganosiloxanes insolubles dans la composition de l'invention. Elles peuvent se présenter sous forme d'huiles, de cires, de résines ou de gommes.
Les organopolysiloxanes sont définis plus en détail dans l'ouvrage de Walter NOLL "Chemistry and Technology of Silicones" (1968), Academic Press. Elles peuvent être volatiles ou non volatiles.
Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7, de préférence de 4 à 5 atomes de silicium. Il s'agit, par exemple, de l'octaméthylçyclotétrasiloxane commercialisé notamment sous le nom de VOLATILE SILICONE® 7207 par UNION CARBIDE ou SILBIONE® 70045 V 2 par RHODIA, le décaméthylcyclopentasiloxane commercialisé sous le nom de VOLATILE SILICONE® 7158 par UNION CARBIDE, et SILBIONE® 70045 V 5 par RHODIA, ainsi que leurs mélanges.
   On peut également citer les cyclocopolymères du type diméthylsiloxane/méthylalkylsiloxane, tel que la SILICONE VOLATILE® FZ 3109 commercialisée par la société UNION CARBIDE, de formule : 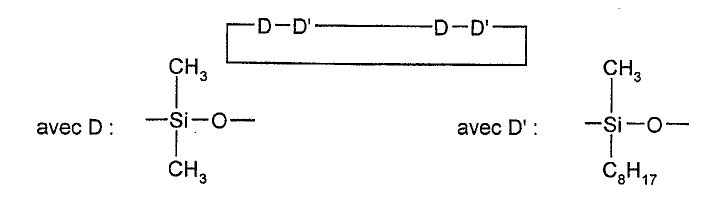
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et présentant une viscosité inférieure ou égale à 5.10⁻⁶m²/s à 25 °C. Il s'agit, par exemple, du décaméthyltétrasiloxane commercialisé notamment sous la dénomination SH 200 par la société TORAY SILICONE. Des silicones entrant dans cette classe sont également décrites dans l'article publié dans *Cosmetics and Toiletries,* Vol. 91, Jan. 76, p. 27-32 - TODD & BYERS *"Volatile Silicone Fluids for Cosmetics".*
On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.
Ces silicones sont plus particulièrement choisies parmi les polyalkylsiloxanes parmi lesquels on peut citer principalement les polydiméthylsiloxanes à groupements terminaux triméthylsilyle. La viscosité des silicones est mesurée à 25°C selon la norme ASTM 445 Appendice C.
Parmi ces polyalkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple, l'huile 70 047 V 500 000 ;
- les huiles de la série MIRASIL® commercialisées par la société RHODIA ;
- les huiles de la série 200 de la société DOW CORNING telles que la DC200 ayant une viscosité de 60 000 mm²/s ;
- les huiles VISCASIL® de GENERAL ELECTRIC et certaines huiles des séries SF (SF 96, SF 18) de GENERAL ELECTRIC.
On peut également citer les polydiméthylsiloxanes à groupements terminaux diméthylsilanol, connus sous le nom de dimethiconol (CTFA), tels que les huiles de la série 48 de la société RHODIA .
Dans cette classe de polyalkylsiloxanes, on peut également citer les produits commercialisés sous les dénominations ABIL WAX® 9800 et 9801 par la société GOLDSCHMIDT qui sont des polyalkyl(C₁-C₂₀)siloxanes.
Les polyalkylarylsiloxanes sont particulièrement choisis parmi les polydiméthyl/méthylphénylsiloxanes, les polydiméthyldiphényl-siloxanes linéaires et/ou ramifiés de viscosité allant de 1.10⁻⁵ à 5.10⁻² m²/s à 25°C.
Parmi ces polyalkylarylsiloxanes on peut citer, à titre d'exemple, les produits commercialisés sous les dénominations suivantes :
- les huiles SILBIONE® de la série 70 641 de RHODIA ;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.
On peut plus particulièrement citer les produits suivants :
- polydiméthylsiloxane
- les gommes polydiméthylsiloxane/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA), et d'un polydiméthylsiloxane cyclique, également appelé cyclométhicone (CTFA), tel que le produit Q2 1401 commercialisé par la société DOW CORNING ;
- les mélanges d'une gomme polydiméthyl-siloxane et d'une silicone cyclique, tels que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶ m²/s. Ce produit comporte de préférence 15 % de gomme SE 30 et 85 % d'une huile SF 96.
   Les résines d'organopolysiloxanes utilisables conformément à l'invention sont des systèmes siloxaniques réticulés renfermant les motifs :
   R₂SiO_{2/2}, R₃SiO_{1/2}, RSiO_{3/2} et SiO_{4/2}
dans lesquels R représente un groupement hydrocarboné possédant 1 à 16 atomes de carbone ou un groupement phényle. Parmi ces produits, ceux particulièrement préférés sont ceux dans lesquels R désigne un groupe alkyle inférieur en C₁-C₄, plus particulièrement méthyle, ou un groupe phényle.
On peut citer parmi ces résines le produit commercialisé sous la dénomination DOW CORNING 593 ou ceux commercialisés sous les dénominations SILICONE FLUID SS 4230 et SS 4267 par la société GENERAL ELECTRIC et qui sont des silicones de structure diméthyl/triméthylsiloxane.
On peut également citer les résines du type triméthylsiloxysilicate commercialisées notamment sous les dénominations X22-4914, X21-5034 et X21-5037 par la société SHIN-ETSU.
Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.
Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés, substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols, comme les produits commercialisés sous les dénominations GP 72 A et GP 71 de GENESEE ;
- des groupements alcoxylés, comme le produit commercialisé sous la dénomination SILICONE COPOLYMER F-755 par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334;
- des groupements acyloxyalkyle tels que, par exemple, les polyorganosiloxanes décrits dans le brevet US-A-4957732 ;
- des groupements anioniques du type carboxylique comme, par exemple, dans les produits décrits dans le brevet EP 186 507 de la société CHISSO CORPORATION, ou de type alkyl-carboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations ABIL® S201 et ABIL® S255.
- des groupements hydroxyacylamino, comme les polyorganosiloxanes décrits dans la demande EP 342 834. On peut citer, par exemple, le produit Q2-8413 de la société DOW CORNING.

Les silicones telles que décrites ci-dessus peuvent être utilisées seules ou en mélange, en une quantité comprise entre 0,01 et 20 % en poids, de préférence entre 0,1 et 5 % en poids.

Le milieu aqueux cosmétiquement acceptable peut contenir des électrolytes minéraux ou organiques.
Les électrolytes utilisés sont de préférence des sels hydrosolubles minéraux tels que les sels de métaux alcalins, alcalino-terreux, ou d'aluminium d'acide chlorhydrique, sulfurique ou nitrique, ou bien des sels d'acides organiques tels que les carbonates, lactates, citrates ou tartrates de métaux alcalins ou alcalino-terreux ou d'aluminium. Les électrolytes particulièrement préférés sont choisis parmi le sulfate de potassium, le sulfate de sodium, le sulfate de magnésium, le nitrate de calcium, le nitrate de magnésium, le chlorure de sodium, le chlorure de potassium, le carbonate de potassium, le carbonate de sodium et le citrate de sodium.

Ces électrolytes sont présents de préférence dans des proportions allant de 0,1 à 30 % en poids, en particulier de 1 à 10 % en poids, rapporté au poids total de la composition.
Le pH des compositions aqueuses de la présente invention est de préférence fixé à une valeur comprise entre 3 et 11, en particulier entre 4 et 9.

### Exemple 1

### Préparation d'un polyuréthanne auto-adhésif

On introduit dans un réacteur thermostaté muni d'un système d'agitation mécanique et d'un réfrigérant les monomères et solvants suivants :
- 1 mole d'un mélange de diols, c'est-à-dire d'un mélange de N-méthyldiéthanolamine et de poly(tétraméthylène oxyde) de masse molaire moyenne en poids égale à 1400, le rapport molaire de la N-méthyldiéthanolamine (NMDEA) au poly(tétraméthylène oxyde) (PTMO) étant égal à 2, et
- une quantité de méthyléthylcétone (solvant) telle que la concentration en monomères diols soit égale à 75 % en poids.

On chauffe le mélange sous agitation jusqu'à une température de 70 °C, puis on introduit goutte-à-goutte sous agitation, sur une durée d'environ 2 heures, un faible excès molaire, c'est-à-dire 1,03 mole de tétraméthylxylylènediisocyanate (OCN-C(CH₃)₂-phénylène-C(CH₃)₂NCO) (TXDI).
Lors de cette addition, on observe une augmentation de la température jusqu'au reflux du solvant.
On prélève à des intervalles réguliers un échantillon dont on trace un spectre d'absorption IR pour suivre la disparition de la bande correspondant aux fonctions isocyanate (2260 cm⁻¹).
Lorsque la bande d'absorption des fonctions -NCO ne diminue plus, ce qui est généralement le cas au bout d'environ 5 heures, on laisse refroidir le mélange réactionnel jusqu'à température ambiante, puis on dilue avec de l'acétone jusqu'à une concentration en polymère d'environ 40 % en poids.
On ajoute ensuite au mélange obtenu 20 ml d'éthanol afin de désactiver les fonctions -NCO résiduelles et l'on poursuit l'agitation à température ambiante jusqu'à disparition totale des fonctions -NCO, c'est-à-dire de la bande d'absorption IR à 2260 cm⁻¹.
On ajoute une solution d'acide chlorhydrique (2 mole/l) en une quantité telle que l'on neutralise 100 % les groupements amine. On élimine ensuite les différents solvants organiques (méthyléthylcétone, acétone et éthanol) par distillation sous vide à une température de 40 °C.
Après élimination de la phase organique, on ajoute à la solution aqueuse du polymère une quantité d'eau suffisante pour obtenir une concentration de polymère dans l'eau d'environ 25 % en poids.

Le polyuréthanne (TXDI/NMDEA/PTMO) ainsi obtenu a une masse molaire moyenne en poids et en nombre, déterminées par chromatographie de perméation de gel, respectivement égales à 70900 et 43800, ce qui permet de calculer un indice de polydispersité d'environ 1,6.

### Exemple 2

On prépare un shampooing de composition suivante :

| | |
|---|---|
| Lauryléthersulfate de sodium à 2,2 moles d'oxydes d'éthylène | 12,5 g (m.a.) |
| Cocoylbétaïne | 2,5 g (m.a.) |
| Polyuréthanne de l'Exemple 1 | 3g (m.a.) |
| Agent d'ajustement de pH | qsq pH 7 |
| Eau déminéralisée | 100 g |

Cette composition confère aux cheveux séchés un effet coiffant qui se traduit par une bonne facilité de mise en forme.

## Revendications

1. Polyuréthannes cationiques ou amphotères portant au moins une fonction amine tertiaire ou quaternaire, **caractérisés par le fait qu'**ils présentent une auto-adhésivité, exprimée par la force de traction maximale (Fₘₐₓ (en N)), supérieure ou égale à 11 N.

2. Polyuréthannes selon la revendication 1, **caractérisés par le fait qu'**ils comprennent
(a) des motifs dérivés d'une ou de plusieurs amines tertiaires ou quaternaires comportant deux fonctions réactives à hydrogène labile,
(b) des motifs dérivés d'un ou de plusieurs polymères non ioniques comportant deux fonctions réactives à hydrogène labile, et
(c) des motifs dérivés d'un ou de plusieurs diisocyanates.

3. Polyuréthannes selon la revendication 2, **caractérisés par le fait que** les motifs (a) sont dérivés d'une ou de plusieurs amines correspondant à l'une des six formules suivantes : 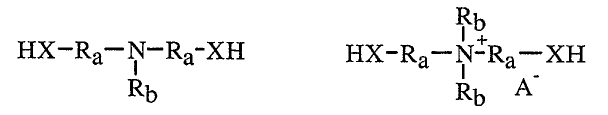 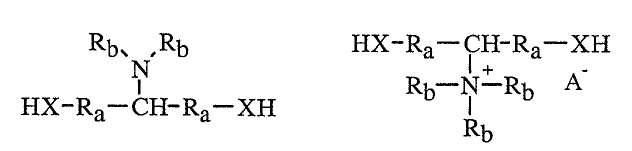 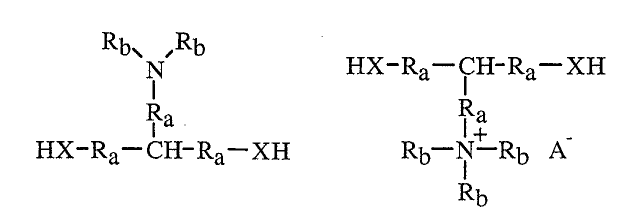 dans lesquelles
chaque Rₐ représente indépendamment un groupe alkylène en C₁₋₆, linéaire ou ramifié, cycloalkylène en C₃₋₆ ou arylène, tous pouvant être substitués par un ou plusieurs atomes d'halogène et comporter un ou plusieurs hétéroatomes choisis parmi O, N, P et S,
chaque R_{b} représente indépendamment un groupe alkyle en C₁₋₆, cycloalkyle en C₃₋₆ ou aryle, tous pouvant être substitués par un ou plusieurs atomes d'halogène et comporter un ou plusieurs hétéroatomes choisis parmi O, N, P et S,
chaque X représente indépendamment un atome d'oxygène ou de soufre ou un groupe NH ou NR_{c}, où R_{c} représente un groupe alkyle en C₁₋₆, et
A⁻ représente un contre-ion physiologiquement acceptable.

4. Polyuréthannes selon la revendication 3, **caractérisés par le fait que** les motifs (a) sont dérivés de la N-méthyldiéthanolamine ou de la N-tert-butyldiéthanolamine.

5. Polyuréthannes selon l'une quelconque des revendications 2 à 4, **caractérisés par le fait que** les motifs (a) à fonction amine tertiaire sont partiellement ou totalement neutralisés par des acides minéraux ou organiques.

6. Polyuréthannes selon l'une quelconque des revendications 2 à 5, **caractérisés par le fait que** les polymères formant les motifs (b) sont choisis parmi les polyéthers, les polyesters, les polysiloxanes, les copolymères d'éthylène et de butylène, les polycarbonates ou les polymères fluorés ayant tous une température de transition vitreuse inférieure à 0 °C.

7. Polyuréthannes selon la revendication 6, **caractérisés par le fait que** les polymères formant les motifs (b) ont une masse molaire moyenne en poids comprise entre 400 et 10 000, de préférence entre 500 et 5000.

8. Polyuréthannes selon la revendication 6 ou 7, **caractérisés par le fait que** les motifs (b) sont dérivés de poly(tétraméthylène oxyde).

9. Polyuréthannes selon l'une quelconque des revendications 2 à 8, **caractérisés par le fait que** les motifs (c) sont dérivés de diisocyanates choisis parmi le tétraméthylxylylènediisocyanate, le méthylènediphényldiisocyanate, le méthylènecyclohexanediisocyanate, l'isophoronediisocyanate, le toluènediisocyanate, le naphtalènediisocyanate, le butanediisocyanate et l'hexyldiisocyanate.

10. Polyuréthannes selon l'une quelconque des revendications précédentes, **caractérisés par le fait que** le rapport molaire des motifs (a) aux motifs (b) est compris entre 0,01 et 50, de préférence entre 0,1 et 6, mieux encore entre 0,2 et 5 et idéalement entre 0,3 et 5.

11. Polyuréthannes selon l'une quelconque des revendications précédentes, **caractérisés par le fait qu'**ils comportent en outre un ou plusieurs motifs anioniques (d).

12. Polyuréthannes selon la revendication 11, **caractérisé par le fait que** les motifs anioniques (d) sont dérivés d'acides carboxyliques ou d'acides sulfoniques comportant deux fonctions à hydrogène labile.

13. Polyuréthannes cationiques selon l'une quelconque des revendications précédentes, **caractérisés par le fait que** le ou les polymères non ioniques formant les motifs (b) présentent une température de transition vitreuse (Tg), déterminée par analyse calorimétrique différentielle, inférieure à 0 °C, de préférence inférieure à -5 °C et en particulier inférieure à -10 °C.

14. Polyuréthannes cationiques selon l'une quelconque des revendications précédentes, **caractérisés par le fait qu'**ils présentent au moins une température de transition vitreuse (Tg), déterminée par analyse calorimétrique différentielle, inférieure à 20 °C, de préférence inférieure à 0 °C et en particulier inférieure à -20 °C.

15. Composition cosmétique contenant, dans un milieu aqueux cosmétiquement acceptable, au moins un polyuréthanne cationique ou amphotère auto-adhésif selon l'une quelconque des revendications précédentes.

16. Composition cosmétique selon la revendication 15, **caractérisée par le fait qu'**elle contient de 0,01 à 40 %, de préférence de 0,05 à 20 % et en particulier de 0,1 à 20 % en poids de polyuréthannes cationiques selon l'une des revendications 1 à 13.

17. Composition cosmétique selon l'une des revendications 15 et 16, **caractérisée par le fait qu'**il s'agit d'une composition de soin, de conditionnement, de maquillage ou de fixation des matières kératiniques humaines, en particulier des cheveux et des cils.

18. Composition cosmétique selon la revendication 17, **caractérisée par le fait qu'**il s'agit d'une composition de coiffage.

19. Composition cosmétique selon la revendication 18, **caractérisée par le fait qu'**il s'agit d'une composition de coiffage à rincer.

20. Composition cosmétique selon l'une quelconque des revendications 15 à 19, **caractérisée par le fait qu'**elle contient des additifs choisis parmi les agents tensioactifs, des polymères anioniques, amphotères, zwitterioniques ou non ioniques, des polymères cationiques différents des polyuréthannes cationiques selon les revendications 1 à 14, des agents nacrants et/ou opacifiants, des solvants organiques, des parfums, des huiles minérales, végétales et/ou synthétiques, des esters d'acides gras, des pigments et colorants, des silicones, des particules minérales ou organiques, des agents de stabilisation du pH, des agents conservateurs et des agents absorbant les UV.

21. Composition cosmétique selon la revendication 20, **caractérisée par le fait que** les agents tensioactifs sont choisis parmi les agents tensioactifs anioniques, non ioniques, amphotères et cationiques et des mélanges de ceux-ci.

22. Composition cosmétique selon la revendication 21, **caractérisée par le fait que** les agents tensioactifs anioniques sont présents à raison de 0,5 à 60 % en poids, de préférence de 5 à 20 % en poids, et que les agents tensioactifs non-ioniques, amphotères et cationiques sont présents à raison de 0,1 % à 30 % en poids, de préférence de 0,5 % à 25 % en poids par rapport au poids total de la composition.

23. Composition cosmétique selon la revendication 20, **caractérisée par le fait que** les silicones sont choisies parmi les silicones volatiles ou non volatiles, cycliques, linéaires ou ramifiées, modifiées ou non par des groupements organiques

24. Composition cosmétique selon la revendication 23, **caractérisée par le fait que** les silicones sont présentes à raison de 0,01 à 20 % en poids, de préférence de 0,1 à 5 % en poids.

25. Utilisation en cosmétique des polyuréthannes cationiques autoadhésifs selon l'une quelconque des revendications 1 à 14.

26. Procédé de traitement des matières kératiniques comprenant l'application d'une composition cosmétique selon l'une quelconque des revendications 15 à 24 sur les matières kératiniques à traiter.

27. Procédé de coiffage comprenant l'application d'une composition cosmétique selon l'une quelconque des revendications 15 à 24 sur les cheveux, le rinçage des cheveux, puis la mise en forme et le séchage des cheveux rincés.
